# EUROPEAN PATENT SPECIFICATION

(11) **EP 2 925 159 B1**
(45) Date of publication and mention of the grant of the patent: **07.11.2018**
(21) Application number: 13859001.3
(22) Date of filing: 26.11.2013
(51) Int. Cl.: A23L 33/21, A23L 33/10

(54) **COMPOSITIONS FOR BALANCING GUT MICROBIOTA AND THE PREPARATION AND THE USES THEREOF**
ZUSAMMENSETZUNGEN ZUM AUSGLEICH DER DARMFLORA UND HERSTELLUNG UND VERWENDUNGEN DAVON
COMPOSITIONS PERMETTANT D'ÉQUILIBRER LA FLORE MICROBIENNE INTESTINALE, LEUR PRÉPARATION ET LEURS UTILISATIONS

(30) Priority: 27.11.2012 CN 201210489456
(43) Date of publication of application: 07.10.2015
(73) Proprietor: Shanghai Jiaotong University, Shanghai 200240 (CN); Perfect (China) Co. Ltd, Guangdong 528420 (CN)
(72) Inventor: ZHAO, Liping, Shanghai 200240 (CN)
(74) Representative: Haseltine Lake LLP
(86) International application number: PCT/CN2013/087813
(87) International publication number: WO 2014/082560

(56) References cited:
- EP-A1- 1 010 374
- EP-B1- 2 498 626
- WO-A1-2013/182038
- CN-A- 101 278 736
- CN-A- 102 273 585
- CN-A- 102 987 383
- GB-A- 2 412 834
- US-A1- 2010 284 972
- I-TE LEE ET AL: "Combined extractives of red yeast rice, bitter gourd, chlorella, soy protein, and licorice improve total cholesterol, low-density lipoprotein cholesterol, and triglyceride in subjects with metabolic syndrome", NUTRITION RESEARCH, ELSEVIER INC, XX, vol. 32, no. 2, 24 December 2011 (2011-12-24), pages 85-92, XP028459243, ISSN: 0271-5317, DOI: 10.1016/J.NUTRES.2011.12.011 [retrieved on 2012-01-03]
- CHUNG-HUANG TSAI ET AL: "Wild bitter gourd improves metabolic syndrome: A preliminary dietary supplementation trial", NUTRITION JOURNAL, BIOMED CENTRAL, GB, vol. 11, no. 1, 13 January 2012 (2012-01-13), page 4, XP021118532, ISSN: 1475-2891, DOI: 10.1186/1475-2891-11-4
- Vidya Gadang ET AL: "Dietary Bitter Melon Seed Increases Peroxisome Proliferator-Activated Receptor-[gamma] Gene Expression in Adipose Tissue, Down-Regulates the Nuclear Factor-[kappa]B Expression, and Alleviates the Symptoms Associated with Metabolic Syndrome", JOURNAL OF MEDICINAL FOOD, vol. 14, no. 1-2, 1 January 2011 (2011-01-01), pages 86-93, XP55367878, US ISSN: 1096-620X, DOI: 10.1089/jmf.2010.0010
- Qixuan Chen ET AL: "Nutrient Metabolism Bitter Melon (Momordica charantia) Reduces Adiposity, Lowers Serum Insulin and Normalizes Glucose Tolerance in Rats Fed a High Fat Diet 1", , 1 January 2003 (2003-01-01), XP55358559, Retrieved from the Internet: URL:http://jn.nutrition.org/content/133/4/ 1088.full.pdf#page=1&view=FitH [retrieved on 2017-03-24]

## Description

### TECHNICAL FIELD

The present application relates to compositions for promoting health. More specifically, the application provides compositions for promoting beneficial gut microbiota or inhibiting opportunistic pathogens, and discloses the preparation and use of the composition.

### BACKGROUND

Unless otherwise indicated herein, the materials described in this section are not prior art to the claims in this application and are not admitted to be prior art by inclusion in this section,

Metabolic syndrome is characterized by, among others, hyperglycemia, hypertension, high cholesterol and overweight. Its most important features are central obesity and insulin resistance. Metabolic syndrome is a high-risk predisposing factor for many chronic diseases such as diabetes, coronary heart disease, cerebral apoplexy and colon cancer, making the metabolic syndrome one of the major health threats. The development of new technologies and methods for treating metabolic syndrome is of vital importance to public health.

Currently, control of metabolic syndrome is mainly through use of drugs and changing the composition of diet. For a long while, because there is no breakthrough in understanding the causes of metabolic syndrome, the use of drugs has been limited to addressing the symptoms instead of the cause of metabolic syndrome. Although the symptoms may be relieved, disease processes are not blocked, and most patients still develop different chronic diseases. Attempts to address metabolic syndrome by changing diet composition does not fare better, because the past efforts generally emphasize the role of a particular nutrient while ignoring the underlying cause of metabolic syndrome. For example, various iterations of low-sugar food to control blood sugar or low-fat food to control blood lipids are popular mainstay, yet success is uncommon, improvement is limited, because the underlying cause of metabolic syndrome is not properly addressed. If diet composition is changed substantially, such as when shifting from high-calorie food of animal origin to plant-based food, or drastic reduction of calorie intake, patients tend to suffer from various adverse reactions, such as unbearable food craving or increased level of inflammatory response, resulting in the failure to strictly follow medical guidance in long term.

GB2412834A describes ready-to-use therapeutic food compositions comprising prebiotics.

CN102273585A describes health-care porridges comprising coarse grains which have the effect of relaxing the bowl.

EP2498626A1 provides nutritional compositions for promoting gut microbiota balance and health.

US2010/284972A1 describes compositions for improving the health of a subject comprising alpha-(1,2)-branched alpha-(1,6) oligodextrans.

EP1010374A1 describes an enteral composition which contains a protein source, a lipid source, a carbohydrate source, and a fibre blend.

Lee et al. (2011) Combined extractives of red yeast rice, bitter gourd, chlorella, soy protein, and licorice improve total cholesterol, low-density lipoprotein cholesterol, and triglyceride in subjects with metabolic syndrome, Nutr. Res., Vol. 32, No. 2, p 85-92, describes plant extractives from red yeast rice, bitter gourd, chlorella, soy protein, and licorice that were effective in reducing total and low-density lipoprotein cholesterol.

Tsai et al. (2012) Wild bitter gourd improves metabolic syndrome: A preliminary dietary supplementation trial, Nutr. J., Vol.11, No. 1, p 4, found that wild bitter gourd improved metabolic syndrome in humans.

Gadang et al. (2011) Dietary Bitter Melon Seed Increases Peroxisome Proliferator-Activated Receptor-[gamma] Gene Expression in Adipose Tissue, Down-Regulates the Nuclear Factor-[kappa]B Expression, and Alleviates the Symptoms Associated with Metabolic Syndrome, J. Med. Food, Vol. 14, No. 1-2, p86-93, found that bitter melon seed improves serum and liver lipid profiles and serum glucose levels.

Chen et al. (2003) Nutrient Metabolism Bitter Melon (Momordica charantia) Reduces Adiposity, Lowers Serum Insulin and Normalizes Glucose Tolerance in Rats Fed a High Fat Diet 1, J. Nutr., Vol. 133, p1088-1093, found that bitter melon reduced adiposity in rats fed a high fat diet.

### SUMMARY

In addition to the illustrative aspects, embodiments, and features described above, further aspects, embodiments, and features will become apparent by reference to the drawings and the following detailed description.

The present application relates to an unexpected discovery that a new composition is effective in promoting beneficial intestinal bacteria while inhibiting opportunistic pathogens, resulting in prevention and/or treatment of metabolic syndrome.

In a first aspect, the invention provides a combination, comprising a first composition, a second composition and a third composition, for use in a method of improving metabolic syndrome in a subject,
wherein the first composition comprises adlay, oat, buckwheat, white bean, yellow corn, red bean, soybean, yam, big jujube, peanut, lotus seed, and wolfberry;
wherein the second composition comprises from 15 to 99.8% of bitter gourd by weight, from 0.1 to 51% of a first fermentable dietary fiber by weight, and from 0.1 to 34% of a first oligosaccharide by weight; and
wherein the third composition comprises a second fermentable dietary fiber and a second oligosaccharide
wherein the intake of the first composition is set to the extent where the subject no longer feels hungry;
wherein the second composition is taken in the form of a granular infusion of 2-3 bags at 20g/bag per day with warm water; and
wherein the daily intake of the third composition is from 30 to 100 grams suspended or dissolved in 800 to 1200ml of water and then consumed by a subject with an empty stomach in the morning.

The first and the second fermentable dietary fiber may or may not be the same. The first and the second oligosaccharide may or may not be the same.

The ingredients in the compositions listed above may include seeds, fruits or other parts or their extracts of a plant that is edible and known or recognized to have medicinal or therapeutic activities.

When ingested by a subject in effective amount, the composition may be capable of increasing a first gut microbiota population including population a short-chain fatty acid (SCFA)-producing bacteria, decreasing a second gut microbiota population including an endotoxin producing bacteria in the subject, or both. For example, the first composition may be capable of increasing the first gut microbiota population, and the second composition may be capable of decreasing the second gut microbiota population in the subject.

The compositions in this application may be food, nutriceutical products, supplements, or healthcare products. The composition may be administered orally to a subject in need thereof. The subject may be a human being. The subject may suffer from metabolic syndrome.

This application discloses methods for making the above compositions. In one example, the method includes mixing adlay, oat, buckwheat, white bean, yellow corn, red bean, soybean, yam, big jujube, peanut, lotus seed, and wolfberry to provide a first composition; mixing bitter gourd, a first fermentable dietary fiber and a first oligosaccharides to provide a second composition; and mixing a second fermentable dietary fiber and a second oligosaccharides to provide a third composition.

The application discloses methods for balancing gut microbiota and/or improving metabolic syndrome in a subject. In one example, the method includes the steps of administering to the subject an effective amount of a treatment composition. The treatment composition may include the above described first composition, the above described second composition, the above described third composition, or any combination of the three compositions.

Compared to existing techniques, the solutions in the present application have, among others, one or more following surprising advantages: when administered to the subject, the compositions are capable of balancing or improve gut microbiota in a subject, improving metabolic syndrome, increasing the amount of short-chain fatty acid-producing bacteria and decreasing the amount of endotoxin-producing bacteria; the compositions may be administered to a subject via various forms and may alleviate, relieve, remedy, prevent or ameliorate metabolic syndrome or symptoms of metabolic syndrome and/or restore health to the subject.

### BRIEF DESCRIPTION OF THE DRAWINGS

The foregoing and other features of this disclosure will become more fully apparent from the following description and appended claims, taken in conjunction with the accompanying drawings. Understanding that these drawings depict only several embodiments arranged in accordance with the disclosure and are, therefore, not to be considered limiting of its scope, the disclosure will be described with additional specificity and detail through use of the accompanying drawings, in which:

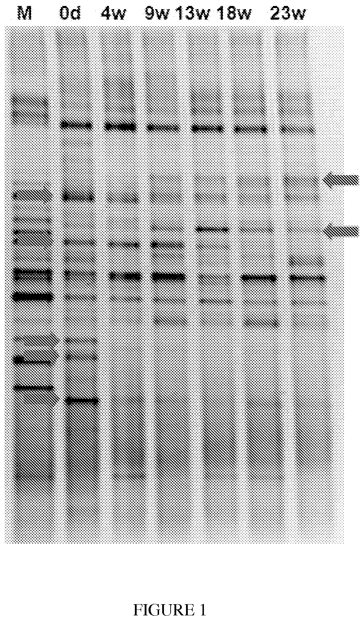

FIGURE 1 shows the fingerprints of the 16S rRNA gene V3-DGGE of the gut microbiota population from the volunteers before and after the intervention.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting.

In one aspect, the application provides novel compositions that are effective in promoting the beneficial gut microbiota population or inhibiting the opportunistic pathogens. The composition may be a nutritional intervention composition. The composition may be made from common food ingredients and may not contain any artificial synthetic colors, hormones, sweeteners, flavoring agents, preservatives, or any illicit drugs, thus is safe and nontoxic. The composition overcomes the insufficiency of the existing nutritional intervention methods for treating metabolic syndrome, and is effective in inhibiting intestinal opportunistic pathogens, increasing the number of beneficial bacteria, reducing chronic inflammation, thus fundamentally improving metabolic syndrome symptoms.

The first composition may be capable of increasing short-chain fatty acid (SCFA)-producing bacteria population in a subject. The first composition comprises adlay (also known as coix seed or Yi Ren), oat, buckwheat, white bean, yellow corn, red bean, soybean, yam, big jujube, peanut, lotus seed, and wolfberry. The first composition may further include whole cereals, beans, nuts, corn (including, for example, white corn, red corn, purple corn, black corn, bi-color corn, multi-color corn), cloves, star anise, fennel, thistle, hawthorn, purslane, *Zaocys* dhumnades, plum, papaya, hemp seed, odoratum, fragrant *Solomonseal* rhizome, licorice, angelica, ginkgo, white lentils, white lentils flower, longan pulp (also called Gui Yuan), cassia, lily, nutmeg, cinnamon, amla, bergamot, almond (either sweet or bitter), *Hippophae* rhamnoides, oysters, Gorgon fruit, pepper, donkey hide gelatin, inner wall of chicken gizzard, malt, kelp, jujube (including, for example, Zizyphus jujube, sour jujube, or black jujube), Momordica grosvenori, Yu Li seed, honeysuckle flower, olive, *Houttuynia* cordata, ginger (including, for example, *Zingiber officinale,* fresh or dried), Hovenia dulcis Thunb, medlar, gardenia, Amomum villosum, Pang Dahai, Poria, citron, Elsholtzia, peach kernel, mulberry leaf, mulberry, orange, bellflower, *Alpinia* oxyphylla, lotus leaf, semen raphani, Alpinia officinarum hance, *Lophatherum* gracile, fermented soybean, chrysanthemum, chicory, Huang Jiezi, Huang Jing, perilla seed, perilla seed, Ge Gen, black sesame seed, black pepper, Sophora japonica, sophora flower, dandelion, honey, Torreya, semen Ziziphi Spinosae, Rhizoma imperatae, reed rhizome, viper, orange peel, peppermint, Allium macrostemon Bunge, raspberry, ageratum, or any combination thereof.

The beans may include black beans, broad beans, peas, mung bean, long bean, Pigeonpea, four winged bean, chickpea, lentil, Pinto bean, green bean, kidney bean, Navy bean, Pea bean, Lima bean, black bean, Garbanzo peas, black-eyed peas, lablab bean or any combination thereof. In one embodiment, the lablab bean may include white Hyacinth bean and white Hyacinth flowers. In one embodiment, the lablab bean may include bean from a plant of Hyacinthus.

The nuts may include peach seeds, almonds, gingko seeds, or a combination thereof. In one embodiment, the almonds may include sweet almonds or bitter almonds.

The whole cereals may include rice, wheat, barley, quinoa, rye, or triticale. The buckwheat may include common buckwheat, bitter Tartary buckwheat, or a combination thereof. In one embodiment, the buckwheat may include seeds from a plant of Gagopyrum, Erigonum, or Fallopia. The oat may include seeds from a plant of Avena.

In one embodiment, the first composition may include powder or granular particles of the whole cereals. In one embodiment, from about 15 to about 100% of the whole cereal granular particles have a diameter of 0.65mm or above. In some embodiments, at least about 15%, at least about 20%, at least about 25%, at least about 30% or at least about 50% of the granular particles of the whole cereals have diameter of 0.65mm or above.

In one example, , the first composition includes from about 10 to about 30% of adlay by weight, from about 5 to about 30% of oat by weight, from about 5 to about 50% of buckwheat by weight, from about 5 to about 20% of white Hyacinth bean by weight, from about 5 to about 30% of yam by weight, or any combination thereof. In one embodiment, the yam is dried yam.

In one example, the first composition may include adlay, oat, buckwheat, white bean, yellow corn, red bean, soybean, yam, peanut, lotus seed, and wolfberry. For example, the first composition includes about 20% by weight of adlay, about 15% by weight of oat, about 10% by weight of buckwheat, about 10% by weight of white Hyacinth beans, about 5% by weight of yellow corn, about 5% by weight of red bean, about 5% by weight of soybean, about 10% by weight of dried yam, about 5% by weight of big jujube, about 5% by weight of peanut, about 5% by weight of lotus seed, and about 5% by weight of wolfberry.

In one embodiment, the first composition may include adlay, oat, buckwheat, lablab bean, yellow corn, red bean, soybean (or, in one example, soy protein), yam, big jujube, peanut, lotus seed, and wolfberry. For example, the amount of the adlay may be from about 10 to about 30%, such as about 10%, about 15%, about 20%, about 25%, or about 30%, by weight of the first composition. The amount of the oat may be from about 5 to about 25%, such as about 5%, about 10%, 15%, 20%, or 25%, by weight of the first composition. The buckwheat may be from about 1 to about 20%, such as about 1%, 5%, about 10%, about 15%, or about 20%, by weight of the first composition. The amount of the lablab bean may be from about 1 to about 20%, such as about 1%, about 5%, about 10%, about 15%, or about 20%, by weight of the first composition. The amount of the yam may be from about 1 to about 20%, such as about 1%, about 5%, about 10%, about 15%, or about 20%, by weight of the first composition. In some embodiments, the yam may be in dried, frozen, canned, or in extracted form. The amount of wolfberry may be from about 5 to about 25%, such about 5%, about 10%, about 15%, about 20%, or about 25%, of the first composition by weight. The amount of soybean may be from about 1 to about 20%, such as about 1%, about 5%, about 10%, about 15%, or about 20%, of the first composition by weight.

In one embodiment, the amount of the adlay, oat, buckwheat, white bean, and yam in the first composition may be about 20%, about 15%, about 10%, about 10%, and 10% by weight, respectively, of the first composition, and the amount of each of the yellow corn, red bean, soybean, big jujube, peanut, lotus seed, and wolfberry may be about 5% by weight of the first composition.

In one embodiment, the first composition may include, per 100g, Vitamin A from about 3 to about 857 µgRE, Vitamin D from about 0.01 to about 5 µgRE, Vitamin E from about 2 to about 79.09 mg, Vitamin B₁ from about 0.01 to about 1.89mg, Vitamin B₂ from about 0.01mg to about 1.4mg, Vitamin B₆ from about 0.01 to about 1.2mg, Vitamin B₁₂ from about 0.1 to about 2.4mg, Vitamin C from about 1mg to about 1170 mg, Niacin from about 0.5 mgNE to about 28.4 mg, calcium (Ca) from about 60-2458mg, phosphorus (P) from about 200 to about 1893mg, potassium (K) from about 350 to about 1796mg, sodium (Na) from about 8 to about 2200 mg, magnesium (Mg) from about 100 to about 350mg, and iron (Fe) from about 2 to about 20mg.

In one embodiment, the first composition includes from about 5 to about 40% or from about 10 to about 20% of protein by weight, from about 30 to about 80% or from about 50 to about 70% of carbohydrate by weight, from about 0.5 to about 30% or from about 2 to about 15% of fat by weight, from about 0.5 to about 30% or from about 2 to about 15% of dietary fiber by weight, from about 0.1 to about 5% or from about 0.5 to about 1% of vitamin(s) by weight, or from about 0.1 to about 2% or from about 0.8 to about 1.2% of mineral(s) by weight.

In one embodiment, every 100 grams of the first composition may provide a total of from about 300 to about 400 calories. In one embodiment, the amount of protein, carbohydrate, fat, fiber, vitamin, and mineral may be from about 13 to about 15%, from about 60 to about 65%, from about 4 to about 6%, from about 5 to about 7%, from about 0.5 to about 1%, and from about 0.8 to about 1.2% by weight, respectively, of the first composition.

The first composition may be in the form of rice, porridge, gruel, congee, or cooked rice. The starch component in these forms is less likely to degrade after steaming, boiling or other forms of cooking and therefore less likely to elevate blood glucose level.

The second composition may be capable of reducing endotoxin-producing bacteria population in a subject. In one embodiment, the second composition may consist of the bitter gourd, the first fermentable dietary fiber, and the first oligosaccharides. The bitter gourd may include powder of whole fruits from a plant of *Momordica charantia* or its extract. The powder of fruits may be produced by freeze drying or spray drying process. The first fermentable dietary fiber may include Fibersol-2, resistant starch, polydextrose, cellulose, hemicellulose, pectin, gum, or a combination thereof. The first oligosaccharides may include independently fructooligosaccharides, galacto-oligosaccharides, lactulose, xylo Isomaltooligosaccharide, soybean oligosaccharides, oligo glucose, Stachyose, Lactosucrose, or a combination thereof.

The second composition includes from 15 to 99.8% of the bitter gourd by weight, from 0.1 to 51% of the first fermentable dietary fiber by weight, and from 0.1 to 34% of the first oligosaccharide by weight. In one embodiment, the ratio of the bitter gourd and oligosaccharides in the second composition may be from about 10:1 to about 1:1 by weight. In one embodiment, the amount of the bitter gourd may be from 15 to about 55%, such as 15%, about 20%, about 30%, about 35%, about 40%, about 45%, about 50%, or about 55%, of the second composition by weight. In one embodiment, the amount of oligofructose may be from about 10 to about 30%, such as about 10%, about 15%, about 20%, about 25%, or about 30%, of the second composition by weight. In one embodiment, the amount of isomaltooligosaccharide may be from about 5 to about 25%, such as about 5%, about 10%, about 15%, about 20%, or about 25%, of the second composition by weight.

The second composition may be in granular or powder form. For example, the second composition may be a granular infusion and can be dissolved or suspended in water. For example, the bitter gourd may be in the form of powder.

The ingredients in the compositions may be in the form of powder, granule, particles, noodle, sheets, crystalline, paste, or solution. The ingredient powder, such as bitter gourd, may be produced by freeze-drying or spray drying. In one embodiment, the granular particles, noodles, and sheets may be made by pulverizing a mixture of the ingredients into a particle or powdery mixture and forming the particle or powdery mixture into granular particles, noodles, or sheets. In one embodiment, from at least about 1% to at least about 70% of the granular particles in the composition has a diameter of 0.65mm or above.

The compositions in the present application may be used as food products, healthcare products, or nutraceuticals, such as dietary supplements. When ingested by a subject in effective amount, the composition may be capable of increasing a first gut microbiota population containing a short-chain fatty acid (SCFA)-producing bacteria, decreasing a second gut microbiota population containing an endotoxin producing bacteria in the subject, or both.

The composition may be mixed with pharmaceutically acceptable carrier to form pharmaceutical compositions. "Pharmaceutically acceptable carriers" include solvents, dispersants, coatings, fillers, adhesives, bulking agents, biologically active agents such as antibacterial or antifungal agents, and isotonic and delayed absorption agents, which are suitable for drug delivery.

The composition may be formulated to be compatible with its intended route of administration. (See, for example, U.S. Patent No. 6,756,196.) Examples of routes of administration include oral, sublingual, buccal, and rectal administration, as well as parenteral administration. For instance, a composition of the present application may be in the form of tablet, capsule, pill, bar, granule, powder, film, microcapsule, aerosol, gruel, congee, porridge, rice or cooked rice, spirit, tincture, tonic, liquid suspension, or syrup. It is advantageous to formulate oral compositions in dosage unit form for ease of administration and uniformity of dosage. "Dosage unit form," as used herein, refers to physically discrete units suited as unitary dosages for the subject to be treated, each unit containing a predetermined quantity of active ingredients calculated to produce the desired therapeutic effect in association with the required pharmaceutical carrier.

The composition of the present application can be used to promote beneficial intestinal bacteria and inhibit opportunistic pathogens. Therefore, the application discloses systems for nutritional intervention for reducing bodyweight or improving metabolic syndrome in a subject. The system may include the above compositions and an instruction configured to instruct the subject on how to use the first composition, the second composition, and the third composition.

For example, an effective amount of the composition may be administered to a subject in need thereof via a proper route such as those described above.

As used herein, a "subject" refers to a human or animal, including all mammals such as primates (particularly higher primates), sheep, dog, rodents (e.g., mouse or rat), guinea pig, goat, pig, cat, rabbit, and cow. In a preferred embodiment, the subject is a human. In another embodiment, the subject is an experimental animal or animal suitable as a disease model.

A subject to be treated may be identified in the judgment of the subject or a health care professional, and can be subjective (e.g., opinion) or objective (e.g., measurable by a test or diagnostic method). For example, a subject to be treated may have a low level of beneficial intestinal bacteria and a high level of opportunistic pathogens, or has been diagnosed with metabolic syndrome.

A "treatment" is defined as administration of a substance to a subject with the purpose to cure, alleviate, relieve, remedy, prevent, or ameliorate a disorder, symptoms of the disorder, a disease state secondary to the disorder, or predisposition toward the disorder.

An "effective amount" is an amount of a composition that is capable of producing a medically desirable result in a treated subject. The medically desirable result may be objective (i.e., measurable by some test or marker) or subjective (i.e., subject gives an indication of or feels an effect).

The dosage required for treating a subject depends on the choice of the route of administration, the nature of the formulation, the nature of the subject's illness, the subject's size, weight, surface area, age, gender, other drugs being administered, and/or the judgment of a health care professional.

The application further discloses instructions for taking the above compositions. In one example, the subject is instructed to take the first composition as a main course of food, take the second composition from about 0.25 to about 1 hour before meal, and take the third composition from about 2 to about 5 hours before meal or with breakfast.

In one embodiment, the first composition may be in the form of a porridge, a gruel, or a congee, and used as food by a subject, optionally in combination with fresh vegetables. The intake amount of the first composition is set to the extent where the subject no longer feels hungry. The second composition is in the form of a granular infusion. The granular infusion may be suspended or dissolved in warm water and consumed by a subject. The daily intake amount of the second composition is from 40 to 60 grams. For example, the subject may be instructed to take 2-3 bags at 20g/bag per day dosage of the second composition with warm water. The third composition may also be a granular infusion. The daily intake amount of the third composition is from 30 to 100 grams. The third composition is suspended or dissolved in 800 to 1200 ml of water and then consumed by a subject with an empty stomach in the morning.

The representative administration cycle of the composition of the present application is from one week to several months, such as one week, two weeks, one month, two months, four months or eight months. When the level of beneficial gut microbiota in the subject's body begins to rise and the level of opportunistic pathogens begins to decline, the dosage of the composition may be reduced gradually. When the composition of the gut microbiota is restored to normal, the administration may be terminated.

The following examples are for illustration of the present application. The embodiments were implemented under the premise of the technical solution of the present application. The detailed implementation methods and specific operation processes are provided. These examples are not intended to limit the scope of the application.

### EXAMPLE 1

### Effective Treatment of an Obese Volunteer by an Example Composition

The composition embodying the present application in a granular infusion form was administered to a volunteer with Body Mass Index (BMI) of 58.78 on a daily basis for 24 weeks. The physiological and biochemical indicators of the body and the changes in the gut microbiota during the administration were systematically monitored. At the end of the intervention, the opportunistic pathogens in the intestine declined; the beneficial bacteria in the intestine increased; intestinal endotoxins entering the host's circulatory system were reduced, the inflammatory response level in the volunteer was lowered; each and every parameter of the metabolic syndrome of the volunteer was significantly improved; and BMI was dropped to 41.50.

### (1) The physical conditions of the volunteer before intervention

The volunteer was a 26-year-old male, having a height of 172.5 cm, a weight of 174.9 kg, a BMI of 58.78, and a waistline of 156.1 cm. According to the BMI evaluation criteria for Asian adults, this volunteer was a severely obese patient. After a systematic physical examination, this volunteer was diagnosed with severe metabolic syndrome. His physiological and biochemical indicators are shown in Table 1 below.

### (2) The composition used for intervention and treatment of the patient

For intervention and treatment of the patient, a granular infusion form of an example composition was used. The example composition has the following formula: the amount of bitter gourd powder was 35%, the amount of wolfberry powder was 15%, the amount of soy protein powder was 10%, the amount of oligofructose was 20%, the amount of isomaltooligosaccharide was 15%, and the amount of wheat fiber was 5%. This composition had good inhibitory effects for intestinal opportunistic pathogens, and was capable of specifically conditioning the contents of the intestinal flora, as demonstrated by the results obtained.

The granular infusion was taken orally 2-3 bags per day (20g/bag), after dissolving in warm water.

### (3) Use of other intervention food

A. Food-like Composition No. 1. The Food-like Composition No. 1 is an example composition in porridge form. The composition has the ingredients including adlay, oat, buckwheat, white bean, yellow corn, red bean, soybean, yam, big jujube, peanut, lotus seed, and wolfberry. This food-like composition was a whole-grain product, with balanced protein, carbohydrates and fat. It was rich in dietary fiber. It was capable of supplementing the dietary and nutritional need of human body such as vitamins and minerals.
   The patient consumed the Food Composition No. 1 as staple food, together with a suitable amount of fresh vegetables during each meal. The food intake was not restricted, and the patient was at liberty to consume the food until he no long felt hungry.
B. Food-like Composition No. 3: The Food-like Composition No. 3 is an example composition in granular infusion form. The composition has the ingredients including oligomeric factor and fermentable dietary fiber. This food-like composition was capable of clearing the intestinal tract, excluding stool, promoting beneficial intestinal bacteria, increasing the production of gas by the beneficial intestinal bacteria, speeding up intestinal motility and emptying, and removing pathogenic bacteria and endotoxin producing bacteria.

This food-like composition contained 30-100 grams of oligosaccharides. The patient consumed this food-like composition every morning prior to other food, along with 800-1200 ml of water.

### (4) Evaluation of the changes in the physiological and biochemical indicators of the body and the effects of the intervention

TABLE 1 show the physiological and biochemical indicators of the volunteer in example I after 9 weeks and 23 Weeks of Intervention

**TABLE 1**

| Item | Measurements | | | Normal range |
|---|---|---|---|---|
| | 0 days before intervention | 9 weeks after intervention | 23 weeks after intervention | |
| Weight (kg) | 174.9 | 144.8 | 123.5 | - |
| BMI | 58.78 | 48.66 | 41.50 | 18-23 |
| fasting blood glucose-FBG (mmol/L) | 8.95 | 4.76 | 5.40 | 3.90-6.10 |
| fasting insulin FINS (µIU/ml) | 58.7 | 25.8 | 23 | 6-27 |
| glycated hemoglobin (%) | 7.58 | 5.44 | 4.52 | 3.8-5.8 |
| insulin resistance indicators | 23.35 | 5.46 | 5.52 | |
| triglycerides (mmol/L) | 2.68 | 1.72 | 1.18 | 0-1.7 |
| total cholesterol (mmol/L) | 5.53 | 4.64 | 4.78 | 3.00-5.17 |
| high density lipoprotein - HDL (mmol/L) | 0.89 | 0.70 | 0.82 | >0.91 |
| low-density lipoprotein-LDL (mmol/L) | 3.42 | 3.15 | 3.42 | 0-4.16 |
| AST (U/L) | 122.0 | 51 | 31 | 10-47 |
| alanine aminotransferase (U/L) | 97.0 | 50 | 33 | 0-41 |
| gamma glutamyl transferase (U/L) | 168.0 | 49 | 59 | 0-56 |
| fatty liver disease-FLD | Severe | Severe | Moderate | Normal |
| systolic pressure-SBP (mmHg) | 150.0 | 120 | 120 | ≤140 |
| diastolic pressure-DBP (mmHg) | 110.0 | 80 | 75 | ≤90 |
| C reactive protein (mg/L) | 14.1 | 9.4 | 9.51 | 0-10 |
| lipopolysaccharide endotoxin-binding protein (µg/ml) | 7.03 | 2.29 | 4.78 | - |
| interleukin 1β (pg/ml) | 0.12 | 0.14 | 0.09 | - |
| interleukin 6 (pg/ml) | 6.71 | 4.46 | 2.76 | - |
| tumor necrosis factor alpha, TNF (pg/ml) | 1.10 | 2.14 | 1.66 | - |
| adiponectin (µg/ml) | 2.00 | 2.09 | 4.27 | - |

A. The intervention by the composition of the present application significantly reduced BMI and drastically improved the metabolic syndrome. Compared with the volunteer's physiological and biochemical indicators 0 day before the intervention, after 9 weeks and 23 weeks of intervention, the volunteer's weight and BMI dropped significantly; the decrease in the indicators of fasting glucose, fasting insulin, glycated hemoglobin, and insulin resistance directly reflects the significant improvement of the state of glucose homeostasis under the intervention; the decline in triglycerides and total cholesterol indicates the improvement of the body's lipid metabolism; the significant reduction and gradual return to the normal range of aspartate aminotransferase, alanine aminotransferase and γ-glutamyl transferase indicate the mitigation of the liver cell damage; at the same time, both the symptoms of fatty liver disease and the blood pressure were relieved; the decrease of both the inflammatory indicator C-reactive protein and the inflammatory factor interleukin-6 suggests that the level of the inflammatory response caused by the gut microbiota population and having a destructive effect to the human body was reduced under the intervention; the increase of the anti-inflammatory factor adiponectin indicates that the function of the body to eliminate inflammatory response recovered after the intervention; the decrease of the lipopolysaccharide endotoxin binding protein suggests that the level of the endotoxin produced by the bacteria and entering the blood was reduced under the intervention. Taken together, these indicators all show that, after the intervention in the volunteer, each parameter of the metabolic syndrome of the volunteer was significantly improved.
B. The intervention by the composition of the present application significantly changed the composition of the intestinal flora, increasing beneficial bacteria and decreasing harmful bacteria. The changes of the composition of the gut microbiota during the intervention were measured by 16S rRNA gene V3-DGGE fingerprint. As shown in FIGURE 1, the 16S rRNA gene V3-DGGE fingerprint shows that the dietary intervention exerted a great impact on the composition of the gut microbiota in this individual. At several points after the intervention, the gut microbiota of the volunteer was relatively similar, indicating that the dietary intervention significantly changed the composition of the intestinal flora.
C. The results of 454 pyrosequencing reveal a significant decrease in Proteobacteria in the volunteer. In particular, the change in Enterobacteriaceae was the most prominent, from 17.90% at -30d to 0.10% at 9w and 0.10% at 23w. Reflected at the genus level, it was manifested by the significant decline of the opportunistic pathogen *Enterobacter.* In addition, at the genus level, after the dietary intervention, the *Faecalibacterium* went upward. *Faecalibacterium* is anti-inflammatory and produces butyrate. It plays an important role in energy metabolism in a host and the protection of the integrity of the intestinal mucosa.

Thus, after the intervention, the number of the opportunistic pathogens in the volunteer declined. The intestinal endotoxins entering the host's circulatory system were reduced, the inflammatory response level in the host was lowered, and the symptoms of metabolic syndrome in the volunteer were effectively relieved.

### EXAMPLE 2

### Effective Treatment of 89 Obese Volunteers by an Example Composition

The same example composition and the intervention regime as described in EXAMPLE 1 was applied to 89 volunteers who suffered from second degree central obesity. The physiological and biochemical indicators and the changes in the gut microbiota during the intervention were systematically monitored for all 89 volunteers. After administering the composition in a granular infusion form on a daily basis for 24 weeks, the opportunistic pathogens in the intestine declined; the beneficial bacteria in the intestine increased; intestinal endotoxins entering the host's circulatory system were reduced, the inflammatory response level in the volunteer was lowered significantly; each and every parameter of the metabolic syndrome of the volunteer was significantly improved; and BMI were significantly reduced.

### (1) The physical conditions of the volunteer before intervention

89 volunteers suffering from second degree central obesity were recruited for the application.

### (2) The composition used for intervention and treatment of the patient

The same composition as EXAMPLE 1 was used.

### (3) Use of other intervention food-like composition

The same composition as EXAMPLE 1 was used.

### (4) Evaluation of the changes in the physiological and biochemical indicators of the body and the effects of the intervention

The data shown in TABLE 2 below are mean value ± standard deviation, or median (interquartile range). Paired t test (normally distributed data) or nonparametric Wilcoxon test distribution (non-normally distributed data) were analyzed by SPSS 17.0 statistical software. For comparison between week 9 versus -30 days, and week 23 versus -30 days, * denotes P <0.05, ** denotes P <0.01; for comparison between week 9 versus week 23, ^{§} denotes P <0.05, ^{§§} denotes P <0.01.

**TABLE 2 The physiological and biochemical indicators of the volunteers in EXAMPLE 2 during the process of dietary intervention**

| Item | Measurements | | | Normal range |
|---|---|---|---|---|
| | 30 days before intervention | 9 weeks after intervention | 23weeks after intervention | |
| Weight (kg) | 84.1 (76.7-92.1) | 78.8^{**} (71.6-87.6) | 78.25^{**§} (72-87.7) | - |
| BMI | 31.51 (30.31-33.91) | 29.81^{**} (28.73-32.24) | 29.50^{**§} (28.43-31.47) | 18-23 |
| FBG (mmol/L) | 4.90 (4.64-5.28) | (4.46-5.13) | 4.93^{§§} (4.63-5.37) | 3.90-6.10 |
| FINS (µIU/ml) | 11.9 (8.5-17.1) | 10.7^{**} (6.9-14.3) | 9.26^{**§§} (6.10-13.95) | 6-27 |
| Glycated hemoglobin (%) | 4.34 (3.99-4.60) | 4.83^{**} (4.63-5.06) | 4.78^{**} (4.57-5.02) | 3.8-5.8 |
| Insulin resistance indicators | 2.63 (1.80-3.92) | 2.40^{**} (1.47-3.15) | 1.96^{**§} (1.27-3.10) | |
| Triglycerides (mmol/L) | 1.55 (1.09-2.25) | 1.17^{**} (0.86-1.80) | 1.29^{**} (0.79-1.88) | 0-1.7 |
| Total cholesterol (mmol/L) | 4.45±0.77 | 4.13±0.77^{**} | 4.38±0.78^{§§} | 3.00-5.17 |
| HDL (mmol/L) | 1.05±0.19 | 0.98±0.21^{**} | 1.09±0.23^{*§§} | >0.91 |
| LDL (mmol/L) | 2.46±0.87 | 2.47±0.76 | 2.61±0.70^{*§} | 0-4.16 |
| AST (U/L) | 21 (16-29) | 21 (17-27) | 22 (19-25) | 10-47 |
| Alanine aminotransferase (U/L) | 21 (13-36) | 19^{**} (13-28) | 17^{**} (10.5-26) | 0-41 |
| gamma glutamyl transferase (U/L) | 27 (20-36) | 22^{**} (18-32) | 22^{**} (16-31) | 0-56 |
| SBP (mmHg) | 127 (121-135) | 123^{**} (116-131) | 125 (115-133) | ≤140 |
| DBP (mmHg) | 89 (80-97) | 84^{*} (79-95) | 89 (80-95) | ≤90 |
| C reactive protein (mg/L, n=67) | 6.60 (5.10-8.20) | 4.90^{**} (4.20-6.20) | 5.93^{*§} (4.69-6.85) | 0-10 |
| lipopolysaccharide endotoxin-binding protein (µg/ml) | 23.21 (15.54-35.50) | 19.98^{**} (14.15-30.83) | 23.09^{§§} (11.37-37.06) | - |
| Interleukin 1β (pg/ml) | 0.07 (0.03-0.12) | 0.07 (0.05-0.15) | 0.06 (0.03-0.13) | - |
| Interleukin 6 (pg/ml) | 2.28 (1.79-3.12) | 2.02^{*} (1.62-2.62) | 1.69^{**§§} (1.27-2.47) | - |
| Tumor necrosis factor α (pg/ml) | 1.07 (0.87-1.49) | 1.03 (0.81-1.40) | 1.03^{*} (0.80-1.54) | - |
| adiponectin (µg/ml) | 3.57 (2.56-5.22) | 3.82^{**} (2.90-5.90) | 4.20^{**§} (3.00-6.20) | - |
| intestinal permeability | 0.026 (0.020-0.031) | 0.022^{**} (0.019-0.026) | 0.023^{*} (0.019-0.026) | - |

| | | | | |
|---|---|---|---|---|
| Note: For the intestinal permeability test, 89 samples were collected 30 days prior to the intervention and 9 weeks after the intervention. 76 samples were collected 23 weeks after the intervention. | | | | |

A. Compared with the volunteers' physiological and biochemical indicators 30 days prior to the intervention, 9 weeks and 23 weeks after the intervention, the volunteers' body weight and BMI continuously dropped significantly; the decrease in the indicators of fasting glucose, fasting insulin, glycated hemoglobin, and insulin resistance directly reflects the improvement of glucose homeostasis under the intervention; the decline in triglycerides and total cholesterol indicates the improvement of the body's lipid metabolism; the significant reduction of aspartate aminotransferase, alanine aminotransferase and γ-glutamyl transferase indicates the mitigation of the liver cell damage; at the same time, both the fatty liver disease (68.5% of the volunteers' conditions improved under the intervention, among which 18.0% returned to normal) and blood pressure were relieved; the decrease of both the inflammatory indicator C-reactive protein and the inflammatory factors interleukin 6 and tumor necrosis factor -α suggests that the level of the inflammatory response caused by the gut microbiota was reduced under the intervention; the increase of the anti-inflammatory factor adiponectin indicates that the function of the body to eliminate inflammatory response recovered after the intervention; the decrease of the lipopolysaccharide endotoxin binding protein suggests that the level of the endotoxin produced by the bacteria and entering the blood was reduced under the intervention.
B. The intervention by the composition of the present application significantly changed the composition of the intestinal flora. The results of 454 pyrosequencing show that the number of Proteobacteria in the volunteers decreased significantly while the number of Actinomycetes increased significantly after the intervention. Primarily, three families showed obvious changes after the dietary intervention. The numbers of Enterobacteriaceae and Desulfovibrio dropped significantly, while the number of Bifidobacteria increased significantly.

After the intervention, the numbers of two types of common pathogens, Enterobacteriaceae and Desulfovibrio, in the volunteers declined significantly, while the number of Bifidobacteria, which can protect the intestinal barrier, increased significantly. The intestinal permeability was lowered, and the intestinal endotoxins entering the host's circulatory system were reduced, resulting in the decrease of the inflammatory response level in the host and relief of the symptoms of metabolic syndrome.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations).

Furthermore, in those instances where a convention analogous to "at least one of A, B, and C, etc." is used, in general such a construction is intended in the sense one having skill in the art would understand the convention (e.g., " a system having at least one of A, B, and C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group. As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. For example, a group having 1-3 cells refers to groups having 1, 2, or 3 cells. Similarly, a group having 1-5 cells refers to groups having 1, 2, 3, 4, or 5 cells, and so forth. While various aspects and embodiments have been disclosed herein, other aspects and embodiments will be apparent to those skilled in the art.

## Claims

1. A combination, comprising a first composition, a second composition and a third composition, for use in a method of improving metabolic syndrome in a subject,
wherein the first composition comprises adlay, oat, buckwheat, white bean, yellow corn, red bean, soybean, yam, big jujube, peanut, lotus seed, and wolfberry;
wherein the second composition comprises from 15 to 99.8% of bitter gourd by weight, from 0.1 to 51% of a first fermentable dietary fiber by weight, and from 0.1 to 34% of a first oligosaccharide by weight;
wherein the third composition comprises a second fermentable dietary fiber and a second oligosaccharide
wherein the intake of the first composition is set to the extent where the subject no longer feels hungry;
wherein the second composition is taken in the form of a granular infusion of 2-3 bags at 20g/bag per day with warm water; and
wherein the daily intake of the third composition is from 30 to 100 grams suspended or dissolved in 800 to 1200ml of water and then consumed by a subject with an empty stomach in the morning.

2. The combination for use according to Claim 1, wherein:
(i) the bitter gourd comprises powder of whole fruits from a plant of *Momordica,* or
(ii) the bitter gourd comprises extracts of whole fruits from a plant of *Momordica,* or
(iii) the first or the second fermentable dietary fiber comprises independently resistant starch, polydextrose, cellulose, hemicellulose, pectin, gum, or a combination thereof, or
(iv) the first or the second oligosaccharides comprises independently fructooligosaccharides, galacto-oligosaccharides, lactulose, xyloIsomaltooligosaccharide, soybean oligosaccharides, oligoglucose, Stachyose, Lactosucrose, or a combination thereof.

3. The combination for use according to Claim 2, wherein:
(i) the buckwheat comprises common buckwheat, bitter Tartary buckwheat, or a combination thereof, or
(ii) the buckwheat comprises seeds from a plant of Gagopyrum, Erigonum, or Fallopia, or
(iii) the oat comprises seeds from a plant of Avena.

4. The combination for use according to Claim 1, wherein the first composition comprises, per 100g, Vitamin A from 3 to 857µgRE, Vitamin D from 0.01 to 5 µgRE, Vitamin E from 2 to 79.09 mg, Vitamin B₁ from 0.01 to 1.89mg, Vitamin B2 from 0.01mg to 1.4mg, Vitamin B₆ from 0.01 to 1.2mg, Vitamin B₁₂ from 0.1 to 2.4mg, Vitamin C from 1mg to 1170 mg, Niacin from 0.5 mgNE to 28.4 mg, Cafrom 60-2458mg, P from 200 to 1893mg, K from 350 to 1796mg, Na from 8 to 2200 mg, Mg from 100 to 350mg, and Fefrom 2 to 20mg.

5. The combination for use according to Claim 1, wherein the second composition is in powder form.

6. The combination for use according to Claim 1, wherein the first composition comprises from 10 to 20% of protein by weight, from 2 to 15% of fat by weight, from 50 to 70% of carbohydrate by weight, and from 2 to 15 % of fermentable dietary fiber by weight.

7. The combination for use according to any one of claims 1 to 6, the method comprising
providing the subject the combination of any one of claims 1 to 6, and
instructing the subject to take the first composition as a main course of food, take the second composition from 0.25 to 1 hour before meal, and take the third composition from 2 to 5 hours before meal or with breakfast.

## Patentansprüche

1. Zusammensetzung, umfassend eine erste Zusammensetzung, eine zweite Zusammensetzung und eine dritte Zusammensetzung zur Verwendung in einem Verfahren zur Verbesserung des metabolischen Syndroms in einem Patienten, wobei die erste Zusammensetzung Hiobstränengras, Hafer, Buchweizen, weiße Bohnen, gelben Mais, rote Bohnen, Sojabohnen, Yamswurzeln, große indische Brustbeeren, Erdnüsse, Lotussamen und Gemeinen Bocksdorn umfasst;
wobei die zweite Zusammensetzung 15 bis 99,8 Gewichtsprozent Bittergurke, 0,1 bis 51 Gewichtsprozent von ersten fermentierbaren Ballaststoffen und 0,1 bis 34 Gewichtsprozent eines ersten Oligosaccharids umfasst;
wobei die dritte Zusammensetzung aus zweiten fermentierbaren Ballaststoffen und einem zweiten Oligosaccharid besteht;
wobei die Aufnahme der ersten Zusammensetzung so eingestellt ist, dass der Patient nicht mehr hungrig ist;
wobei die zweite Zusammensetzung mit warmem Wasser in Form eines granulösen Aufgusses eingenommen wird (2 bis 3 20-g-Beutel pro Tag); und
wobei die tägliche Aufnahme - 30 bis 100 Gramm - der dritten Zusammensetzung in 800 bis 1200 ml Wasser gelöst und dann von einem Patienten am Morgen auf leeren Magen eingenommen wird.

2. Zusammensetzung zur Verwendung gemäß Anspruch 1, wobei
i) die Bittergurke aus dem Pulver ganzer Früchte der Bittergurkenpflanze besteht oder
ii) die Bittergurke aus dem Extrakt ganzer Früchte der Bittergurkenpflanze besteht oder
(iii) die ersten oder zweiten fermentierbaren Ballaststoffe unabhängig resistente Stärke, Polydextrose, Cellulose, Hemicellulose, Pektin, Gummi oder eine Kombination davon umfasst oder
(iv) das erste oder zweite Oligosaccharid umfasst unabhängig voneinander Fructo-Oligosaccharide, Milch-Oligosaccharide, Lactulose, Xylo-Isomalto-Oligosaccharide, Sojabohnen-Oligosaccharide, Oligoglucose, Stachyose, Lactosucrose oder eine Kombination davon.

3. Zusammensetzung zur Verwendung gemäß Anspruch 2, wobei
(i) der Buchweizen Gemeinen Buchweizen, bitteren Tartarischen Buchweizen oder eine Kombination davon umfasst oder
(ii) der Buchweizen Samen der Echten Buchweizen-, Wilden Buchweizen- oder Kletterknöterich-Pflanze umfasst oder
(ii) der Hafer Samen der Avena-Pflanze umfasst.

4. Zusammensetzung zur Verwendung gemäß Anspruch 1, wobei die erste Zusammensetzung pro 100 g von 3 bis 857 µgRE Vitamin A, von 0,01 bis 5 µgRE Vitamin D, von 2 bis 79,09 mg Vitamin E, von 0,01 bis 1,89 mg Vitamin B₁, von 0,01 mg bis 1,4 mg Vitamin B₂, von 0,01 bis 1,2 mg Vitamin B₆, von 0,1 bis 2,4 mg Vitamin B₁₂, von 1 mg bis 1170 mg Vitamin C, von 0,5 mg bis 28,4 mg Niacin, von 60 bis 2458 mg Ca, von 200 bis 1893 mg P, von 350 bis 1796 mg K, von 8 bis 2200 mg Na, von 100 bis 350 mg Mg und von 2 bis 20 mg Fe enthält.

5. Zusammensetzung zur Verwendung gemäß Anspruch 1, wobei die zweite Zusammensetzung als Pulver vorliegt.

6. Zusammensetzung zur Verwendung gemäß Anspruch 1, wobei die erste Zusammensetzung 10 bis 20 Gewichtsprozent Protein, 2 bis 15 Gewichtsprozent Fett, 50 bis 70 Gewichtsprozent Kohlenhydrate und 2 bis 15 Gewichtsprozent fermentierbare Ballaststoffe enthält.

7. Zusammensetzung zur Verwendung gemäß einem der Ansprüche 1 bis 6, wobei das Verfahren umfasst, dem Patienten die Zusammensetzung gemäß einem der Ansprüche 1 bis 6 zu verabreichen und
den Patienten anzuweisen, die erste Zusammensetzung als Hauptgericht, die zweite Zusammensetzung eine Viertelstunde bis 1 Stunde vor dem Essen und die dritte Zusammensetzung 2 bis 5 Stunden vor dem Essen oder zusammen mit dem Frühstück einzunehmen.

## Revendications

1. Une combinaison comprenant une première composition, une deuxième composition et une troisième composition, à utiliser dans un procédé d'amélioration du syndrome métabolique chez un sujet, dans laquelle la première composition comprend du coïx, de l'avoine, du sarrasin, du haricot blanc, du maïs jaune, du haricot rouge, du soja, de l'igname, du gros jujube, de la cacahouète, des graines de lotus et de l'airelle ;
dans laquelle la deuxième composition comprend de 15 à 99,8 % de gourde amère en poids, de 0,1 à 51 % d'une première fibre alimentaire fermentescible en poids, et de 0,1 à 34 % d'un premier oligosaccharide en poids ;
dans laquelle la troisième composition comprend une deuxième fibre alimentaire fermentescible et un deuxième oligosaccharide
dans laquelle la prise de la première composition est réglée de telle sorte que le sujet ne ressente plus la faim ;
dans laquelle la deuxième composition est prise sous la forme d'une infusion granulaire de 2-3 sacs à raison de 20 g/sac par jour avec de l'eau chaude ; et dans laquelle l'apport quotidien de la troisième composition est de 30 à 100 grammes en suspension ou dissous dans 800 à 1 200 ml d'eau et ensuite consommé par un sujet à jeun le matin.

2. La combinaison à utiliser selon la revendication 1, dans laquelle :
(i) la gourde amère contient de la poudre de fruits entiers provenant d'une plante de *Momordica,* ou
(ii) la gourde amère contient des extraits de fruits entiers d'une plante de *Momordica*, ou
(iii) la première ou la deuxième fibre alimentaire fermentescible comprend indépendamment de l'amidon résistant, du polydextrose, de la cellulose, de l'hémicellulose, de la pectine, de la gomme ou une combinaison de ceux-ci, ou
(iv) le premier ou le second oligosaccharide comprend indépendamment des fructooligosaccharides, des galacto-oligosaccharides, du lactulose, du xyloIsomaltooligosaccharide, des oligosaccharides de soja, des oligoglucoses, du Stachyose, du Lactosaccharose ou une combinaison de ceux-ci.

3. La combinaison à utiliser selon la revendication 2, dans laquelle :
(i) le sarrasin comprend du sarrasin commun, du sarrasin écossais amer ou une combinaison des deux, ou
(ii) le sarrasin comprend des graines provenant d'une plante de Gagopyrum, Erigonum ou Fallopia, ou
(iii) l'avoine comprend des graines provenant d'une plante d'Avena.

4. La combinaison à utiliser selon la revendication 1, dans laquelle la première composition comprend, pour 100 g, de la vitamine A de 3 à 857 µgRE, de la Vitamine D de 0,01 à 5 µgRE, de la Vitamine E de 2 à 79,09 mg, de la Vitamine B₁ de 0,01 à 1,89 mg, de la vitamine B2 de 0,01 à 1,4 mg, de la vitamine B₆ de 0,01 à 1,2 mg, de la vitamine B₁₂ de 0,1 à 2,4 mg, de la vitamine C de 1 à 1170 mg, de la niacine de 0,5 mgNE à 28,4 mg, du Cafrom 60-2458 mg, P de 200 à 1893 mg, K de 350 à 1796 mg, Na de 8 à 2200 mg, Mg de 100 à 350 mg et du Fefrom de 2 à 20 mg.

5. La combinaison à utiliser selon la revendication 1, dans laquelle la deuxième composition est sous forme de poudre.

6. La combinaison à utiliser selon la revendication 1, dans laquelle la première composition comprend de 10 à 20 % de protéines en poids, de 2 à 15 % de matières grasses en poids, de 50 à 70 % de glucides en poids et de 2 à 15 % de fibres alimentaires fermentescibles en poids.

7. La combinaison destinée à utiliser selon l'une quelconque des revendications 1 à 6, le procédé comprenant : fournir au sujet la combinaison de l'une quelconque des revendications 1 à 6, et
instruire le sujet de prendre la première composition comme plat principal, de prendre la deuxième composition de 0,25 à 1 heure avant le repas, et de prendre la troisième composition de 2 à 5 heures avant le repas ou au petit déjeuner.
